# EUROPÄISCHE PATENTSCHRIFT

(11) **EP 2 523 702 B1**
(45) Veröffentlichungstag und Bekanntmachung des Hinweises auf die Patenterteilung: **12.11.2014**
(21) Anmeldenummer: 11704173.1
(22) Anmeldetag: 10.01.2011
(51) Int. Cl.: A61M 1/10, A61M 1/16

(54) **ANORDNUNG MIT EINER BLUTPUMPE UND EINEM GASAUSTAUSCHER ZUR EXTRAKORPORALEN MEMBRANOXYGENIERUNG**
ARRANGEMENT HAVING A BLOOD PUMP AND A GAS EXCHANGER FOR EXTRACORPOREAL MEMBRANE OXYGENATION
AGENCEMENT COMPORTANT UNE POMPE À SANG ET UN ÉCHANGEUR GAZEUX POUR L'OXYGÉNATION DE MEMBRANE EXTRACORPORELLE

(30) Priorität: 13.01.2010 US 335881 P; 13.01.2010 DE 102010004600
(43) Veröffentlichungstag der Anmeldung: 21.11.2012
(73) Patentinhaber: Marseille, Oliver, 52066 Aachen (DE)
(72) Erfinder: Marseille, Oliver, 52066 Aachen (DE)
(74) Vertreter: Castell, Klaus
(86) Internationale Anmeldenummer: PCT/DE2011/000009
(87) Internationale Veröffentlichungsnummer: WO 2011/085714

(56) Entgegenhaltungen:
- WO-A1-2005/028002
- WO-A1-2009/110652
- WO-A2-2004/043517
- CN-A- 1 528 472
- DE-A1-102007 010 112

## Beschreibung

Die Erfindung betrifft eine Anordnung mit einer Blutpumpe und einem Gasaustauscher zur extrakorporalen Membranoxygenierung, siehe z.B. DE 102 007 010 112.

Das Herz als zentrales Organ des Kreislaufsystems ist ein Hohlmuskel mit zwei Kammern, der durch Kontraktion und Erschlaffen das Blut im Kreislauf fördert. Mit seiner linken Kammer (linker Ventrikel) wird das Blut durch die arteriellen Blutgefäße des großen Kreislaufs zu den Blutkapillaren der Körperperipherie gepumpt. Durch die venösen Blutgefäße gelangt das Blut zurück zur rechten Herzkammer (rechter Ventrikel). Von dort wird es durch die Lungenarterien in den Lungenkreislauf (kleiner Kreislauf) zur Lunge gefördert und gelangt über die Lungenvenen wieder zurück zur linken Herzkammer. Der kleine Kreislauf befindend sich im Brustkorb.

Bei Herzerkrankungen können Patienten in eine Situation gelangen, in der eine künstliche Kreislaufunterstützung zur einzig möglichen und damit lebenserhaltenden Therapie wird. Während bei Herzunterstützungssystemen, die die Pumpfunktion der rechten, linken oder beider Herzseiten ersetzen, eine direkte Verbindung zu Blutgefässen im Brustkorb hergestellt werden muss, ermöglichen die ECMO Systeme (extrakorporale Membranoxygenierung) durch Übernahme bzw. Unterstützung der gesamten Funktion des inneren Kreislaufs, d.h. des rechten und linken und der Lunge, eine einfachere Anschlussmöglichkeit.

ECMO Systeme können an peripheren Blutgefässen angeschlossen werden. Dadurch ist die sogenannte Kanulierung einfacher und sicherer und kann auch außerhalb von spezialisierten Kliniken vorgenommen werden. Zusätzlich kann in einem akuten Notfall ein ECMO System wesentlich schneller kanuliert und damit der Patient mit der lebenswichtigen Unterstützung versorgt werden.

ECMO Systeme bestehen aus einer Blutpumpe und einem Oxygenator, der die Lungenfunktion unterstützt und damit im Blut das CO₂ reduziert und das O₂ anreichert.

ECMO Systeme können das Blut parallel zum inneren Kreislauf fördern, indem das Blut einer Vene (venös) entnommen und einer Arterie (arteriell) zugeführt wird. Hierbei fördert die Pumpe das Blut über die arterio-venöse Druckdifferenz und ermöglicht so parallel zum Herzen die Durchblutung der Köperperipherie und damit der lebenswichtigen Organe.

Auch bei Lungenerkrankungen kann der Einsatz eines ECMO Systems die einzige lebenserhaltende Therapieoption sein. Kann die Lunge auch durch künstliche Beatmung ihre Funktion nicht mehr hinreichend erfüllen, leiden durch die fehlende CO₂ Reduzierung und die O₂ Versorgung sämtliche andere Organe und der Patient gelangt in eine lebensbedrohliche Situation.

Bei der Therapie von Lungenerkrankungen mittels ECMO Systemen können diese auch veno-venös an den Patienten angeschlossen werden, da lediglich die Funktion der Lunge übernommen wird.

Gängige ECMO Systeme beinhalten Oxygenatoren, in denen der Gasaustausch mittels Membranfaserbündeln erfolgt. Der Gastransport erfolgt wie auch in der Lunge über das Konzentrationsgefälle zwischen Blut und Sauerstoff, der dem Oxygenator zugeführt wird. Die gängig verwendeten Oxygenatoren in ECMO Systemen sind Herz-Lungen-Maschinen entliehen, wie sie in der Herzchirurgie während der Operation am Herzen zum Einsatz kommen.

Auch die Pumpen der ECMO Systeme sind der Herz-Lungen-Maschine entliehen. Es kommen Kreiselpumpen mit radialer oder diagonaler Bauform zum Einsatz, die über einen Elektromotor angetrieben werden.

In jüngster Zeit werden ECMO Systeme entwickelt, die derartige Kreiselpumpen und Oxygenatoren kombinieren und somit kompaktere Systeme mit geringerem Füllvolumen (Primingvolumen) ermöglichen. Diese Systeme werden ortsfest eingesetzt und können nicht direkt am Patienten betrieben werden, da sie lageabhängig sind und in geeigneten, starren Halterungen fixiert werden müssen.

Wird bei stabiler Herzfunktion lediglich eine Lungenunterstützung benötigt, wird auch ein System eingesetzt, bei dem das Druckgefälle zwischen den arteriellen und venösen Gefäßen ausgenutzt wird um den Oxygenator zu durchströmen. Diese Systeme kommen daher ohne Pumpe aus.

Derzeitige ECMO Systeme können aufgrund der relativ großen körperfremden Oberfläche nur zeitlich limitiert und unter Einsatz von gerinnungshemmenden Medikamenten eingesetzt werden. Trotz der Medikamente neigen die Systeme zur Thrombenbildung und müssen häufig ausgewechselt werden.

Sie sind kompliziert zu bedienen und benötigen in der Regel Fachpersonal, das mit dem Umgang mit Herz-Lungen-Maschinen vertraut ist.

Die Antriebskonsolen der eingesetzten Systeme sind relativ aufwendig und teuer.

Auch wenn ECMO Systeme mittlerweile zum Patiententransport eingesetzt werden, so sind die Antriebe relativ schwer, da sie zum Transport eine netzunabhängige Stromversorgung benötigen um einen autarken Betrieb zu ermöglichen.

Bei den Systemen ohne Pumpe kann es zu Problemen kommen, wenn sich während der Therapie die Herzfunktion verschlechtert.

Der Erfindung liegt die Aufgabe zu Grunde, eine Anordnung mit einer Blutpumpe und einem Gasaustauscher zur extrakorporalen Membranoxygenierung weiterzuentwickeln.

Diese Aufgabe wird mit einer gattungsgemäßen Anordnung wie beansprucht gelöst, bei der die Blutpumpe als pulsatile Blutpumpe ausgebildet ist und mit dem Gasaustauscher im gleichen Gehäuse angeordnet ist. /

Hierbei wird vorzugsweise ein Aufbau gewählt, bei dem die Blutzuleitung direkt und ohne Reservoir mit der pulsatilen Blutpumpe in Verbindung steht. Ein Reservoir, wie bei Herz-Lungen-Maschinen üblich, entfällt, da es durch die pulsatile Blutpumpe durch limitierte Saugdrücke nicht zum kritischen Ansaugen im Blutgefäß kommen kann.

Weiterhin wird eine Anordnung vorgeschlagen, bei der die Blutableitung direkt mit dem Gastauscher in Verbindung steht. Auf einen Filter zwischen Gastauscher und Blutableitung, wie bei Herz-Lungen-Maschinen üblich, wird bewusst verzichtet, da durch den atraumatischen Gesamtaufbau und die pulsierende Blutförderung die Bildung von Emboli verhindert wird und daher ein Zurückhalten derer nicht nötig ist. Die Blutzu- und Ableitung sind vorzugsweise als Kanülen- oder Schlauchanschluss ausgeführt, um direkt entsprechende Kanülen zum Anschluss an den Patienten anschließen zu können und damit die Leitungen möglichst kurz zu halten.

Durch eine derartige Anordnung entsteht ein System zur extrakorporalen Membranoxygenierung, das als kompakte Einheit leicht transportabel und schnell einsetzbar ist. Der Aufbau ermöglicht es mit wenigen und kurzen Leitungen auszukommen, wodurch die Gefahr einer Blutgerinnung an den Oberflächen von Blutleitungen weiter reduziert wird. Es wird daher vorgeschlagen, dass die Blutzuleitung und die Blutableitung jeweils eine Länge von 80 cm oder weniger als Verbindung zu einem Patienten aufweisen. Das System arbeitet lageunabhängig und benötigt keine speziellen Halterungen. Der nicht ortsfeste Betrieb auch direkt am Patienten ist möglich.

Vorteilhaft ist es, wenn eine am Gehäuse angeordnete Blutzuleitung und eine am Gehäuse angeordnete Blutableitung in die gleiche Richtung weisen. Dadurch können besonders kurze Kanülen verwendet werden und das Gehäuse kann möglichst nah am Patienten angeordnet sein.

Eine weitere Verringerung der Länge der Kanülen kann dadurch erzielt werden, dass eine Blutzuleitung und eine Blutableitung an der gleichen Seite des Gehäuses angeordnet sind.

Ein besonders kompakter Aufbau wird dadurch erzielt, dass die pulsatile Blutpumpe in einer axialen Ausrichtung des Gasaustauschers wirkt.

Als bevorzugte Ausführungsvariante wird die pulsatile Blutpumpe radial innerhalb des Gasaustauschers angeordnet.

Kumulativ oder alternativ kann die pulsatile Blutpumpe an einer Stirnseite des Gasaustauschers angeordnet sein.

Pulsatile Blutpumpen oder Blutpumpen nach pulsatilem Wirkprinzip sind Pumpen die nach dem Verdrängerprinzip arbeiten. In der Füllphase gelangt das Blut durch das passive öffnende Einlassventil in die sich expandierende Pumpkammer. In der Auswurfphase wird das Volumen in der Pumpkammer komprimiert und das Blut wird durch das ebenfalls passiv öffnende Auslassventil ausgeworfen.

Nach einem besonders wesentlichen Aspekt der Erfindung wird vorgeschlagen, dass die pulsatile Blutpumpe pneumatisch angetrieben wird. Die pulsatile Blutpumpe kann mit einem Stößel, der auf einen Kolben in einem Zylinder wirkt oder der auf eine Membran wirkt, angetrieben werden. Vorteilhaft ist jedoch, wenn die Blutpumpe mit einem pulsierenden Gasstrom angetrieben wird. Dadurch werden elektrische Komponenten vermieden. Dies ermöglicht es die gesamte Anordnung ohne oder mit minimaler elektrischer Energieversorgung zu betreiben.

Erfindungsgemäß stehen die pulsatile Blutpumpe und der Gausaustauscher mit der gleichen Gasquelle stehen. Dies ermöglicht es, das für den Gasaustauscher benötigte unter Druck stehende Gas auch als Antriebsgas für die Blutpumpe zu verwenden.

Wenn der Gasaustauscher über ein Ventil mit pulsierendem Druckgas in Verbindung steht, reicht eine Leitung mit pulsierendem Druckgas aus, um die Anordnung mit Gasaustauschergas und Antriebsgas für die Pumpe zu versorgen. Es kann der Gasauslass der pulsierenden Blutpumpe oder des pulsierenden Pumpenantriebs mit dem Gasaustauscher in Verbindung stehen

Nach einem besonders wesentlichen Aspekt der Erfindung wird vorgeschlagen, dass die pulsatile Blutpumpe als Ballonpumpe ausgeführt ist. Sie weist einen Ballon und ein Einlass- sowie ein Auslassventil aufweist. Ein flexibler Ballon ist einfach und preiswert zu fertigen und weist auch bei zahlreichen Lastzyklen eine hohe Ausfallsicherheit auf.

Nach einem weiteren besonders wesentlichen Aspekt der Erfindung wird vorgeschlagen, dass die pulsatile Blutpumpe als Membranpumpe ausgeführt ist, deren Membran derart vorgespannt ist, dass ihre passive Lage die der maximalen Pumpenfüllung ist. Dabei wird die Verdrängerpumpe durch eine vorgespannte Membran ausgeführt. Diese muss in ihrer Ausgangslage, also ohne Treibdruck bzw. Kraft durch einen Stößel, die größte Füllung der Pumpenkammer zulassen. Somit kann die Pumpe durch positiven Druck betrieben werden. Ein sonst üblicher Unterdruck wird nicht benötigt.

Ein kompakter Aufbau entsteht, wenn eine Verdrängerpumpe von Membranfaserpaketen umgeben ist.

Der Ballon der Pumpe wird vorzugsweise pneumatisch angetrieben, während die Membran pneumatisch oder mechanisch angetrieben werden kann.

Vorteilhaft ist eine Anordnung in Strömungsrichtung von Einlass, Einlassventil, Pumpkammer, Auslassventil, Gastauscherfasern und Auslass. Alternativ wird eine Anordnung in Strömungsrichtung vorgeschlagen von Einlass, Einlassventil, Pumpkammer, Gastauscherfasern, Auslassventil und Auslass. So kann ggf. der Gastauschprozess durch schwellende Drücke verbessert werden.

Eine vorteilhafte Ausführungsvariante bildet ein ECMO System mit einer zentral angeordneten Ballonpumpe, bei dem ein ringförmiges Faserbündel radial durchströmt wird. Das Längen- zu Durchmesser-Verhältnis des ringförmigen Faserbündels kann kleiner oder größer oder gleich 1:1 radial durchströmt werden.

Eine alternative Ausführungsform sieht eine stirnseitig angebrachte Membranpumpe vor und ein tonnenförmiges Faserbündel, das diagonal durchströmt wird. Dieses tonnenförmige Faserbündel kann mit einem Längen- zu Durchmesser-Verhältnis von kleiner oder größer oder gleich 1:1 diagonal durchströmt werden.

Anstelle einer zentral angeordneten Ballonpumpe kann auch eine stirnseitig angebrachte Membranpumpe vorgesehen sein.

Die Gasaustauschereinheit kann tonnenförmig, quadratisch und/oder flach ausgeführt sein. Als Ventile eignen sich Kugelventile, Kegelventile, Scheibenventile oder Membranventile.

Unterschiedliche erfindungsgemäße Anordnungen sind in den Figuren dargestellt, die als Ausführungsbeispiele dienen. Es zeigt
- Figur 1: ein ECMO System aus Antriebskonsole und Patientensystem mit Bypass zur Versorgung des Oxygenators,
- Figur 2: eine Anordnung gemäß Figur 1 mit zwei Leitungen zwischen Antriebskonsole und Patientensystem,
- Figur 3: eine Anordnung mit einem Überdruckventil zwischen Antriebskonsole und Patientensystem,
- Figur 4: ein Gehäuse mit radial innen liegendem Ballon und zwei radial innen liegenden Ventilen,
- Figur 5: ein Gehäuse gemäß Figur 4 mit nur einem radial innen liegenden Ventil,
- Figur 6: ein Gehäuse mit tonnenförmigen Gastauscherfasern und stirnseitig angeordneter Blutpumpe,
- Figur 7: ein Gehäuse mit ringförmig angeordneten Gastauscherfasern und stirnseitig angeordneter Blutpumpe,
- Figur 8: ein Gehäuse, bei dem Blutpumpe und Gasaustauscher parallel nebeneinander angeordnet sind, und
- Figur 9: ein System mit mechanisch angetriebener Pumpe.

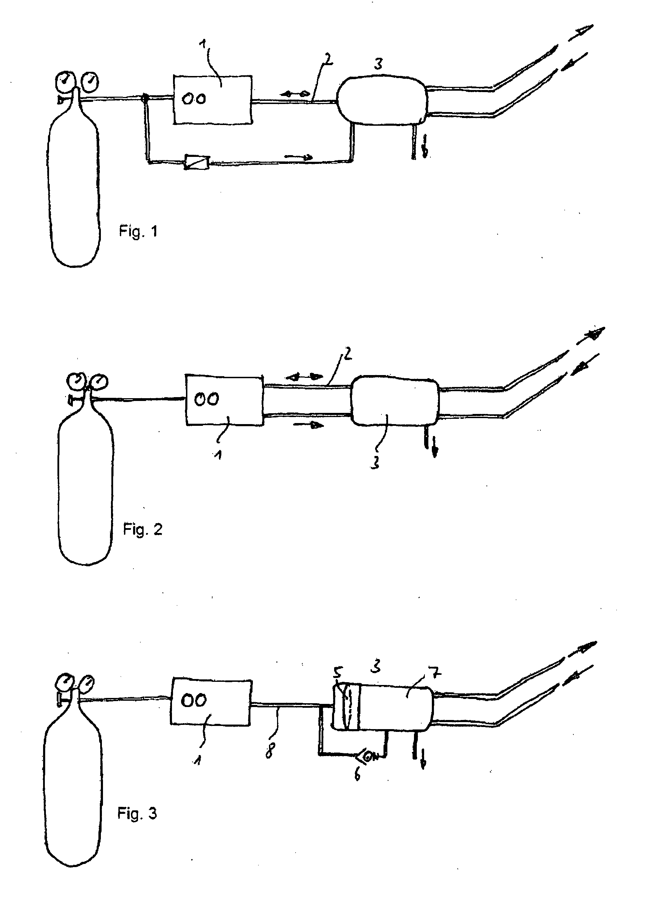

Grundlage der Erfindung ist ein ECMO System, das mit einer pulsatilen Verdrängerpumpe das Blut fördert und bei dem die Antriebsenergie durch das komprimierte Beatmungsgas freigesetzt wird. Das Gas wird hierbei einer pneumatisch arbeitenden Antriebskonsole 1 zugeführt. Die Konsole erzeugt einen wechselnden auf und abschwellenden Druck, der über eine Leitung 2 der Pumpe des Patientensystems 3 zugeführt wird (Fig. 1).

Nachdem das Gas die Konsole durchlaufen hat, kann es anstatt an die Umgebung an den Oxygenator des Patientensystems über eine separate Leitung 4 zugeführt werden (Fig. 2). So wird eine effektivere Ausnutzung des Gases erreicht.

Des weiteren wird eine Lösung vorgeschlagen, bei der wie beschrieben ein auf und abschwellender Druck der Pumpe zugeführt wird und am Patientensystem 3 zur Pumpe 5 ein Überdruckventil 6 parallel geschaltet ist, das auf der anderen Seite mit dem Gastauscher bzw. Oxygenator 7 des Patentensystems verbunden ist und das beim oberen Druckniveau diesen gleichzeitig mit Beatmungsgas versorgt (Fig. 3). Somit ergibt sich lediglich eine Zuleitung 8 zum Patientensystem.

Wie oben beschrieben wird eine pulsatile Blutpumpe mit einem Oxygenator in einer kompakten Einheit, dem Patientensystem, kombiniert. Hierzu werden folgende Prinzipien vorgeschlagen,

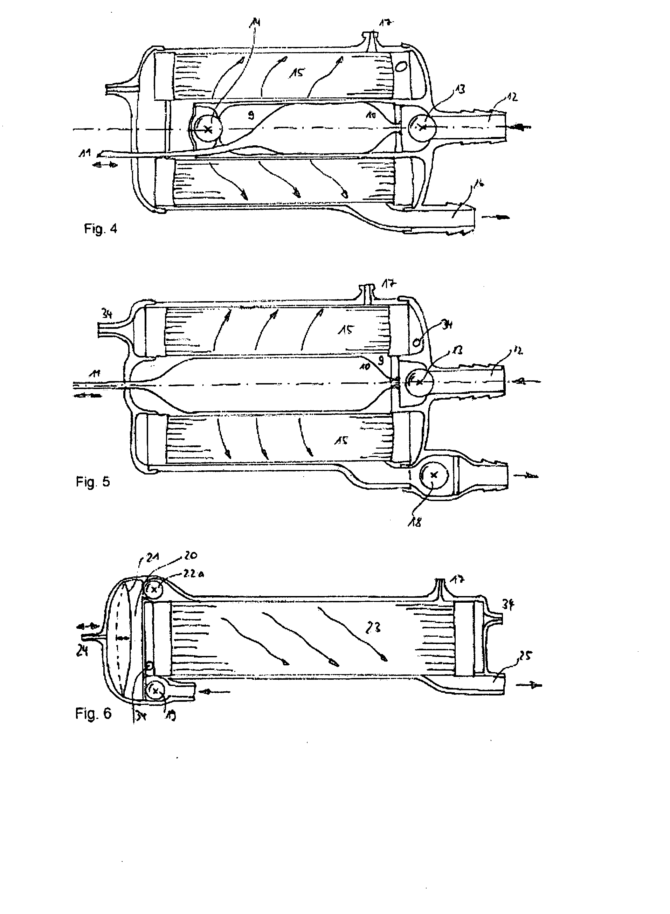

Bei einem Prinzip (Fig. 4) befindet sich in der Pumpkammer 9, die zentral angeordnet ist, ein Ballon 10, der mit einem Anschlussschlauch 11 pulsatil mit Druckluft beaufschlagt und entspannt wird. Das Blut gelangt vom Patienten über einen Stutzen 12 und ein Ventil 13 in die Pumpkammer 9. Durch ein zweites Ventil 14 gelangt das Blut zu den Gastauscherfasern 15, die ringförmig angeordnet sein können. Diese werden radial durchströmt und das Blut gelangt so zum Auslass 16. Gegenüber dem Auslass 16 ist ein Entlüftungsanschluss 17 vorgesehen, um die Befüllung und Entlüftung des Systems zu vereinfachen.

Bei einer alternativen Anordnung (Fig. 5) befindet sich das zweite Ventil 18 in Strömungsrichtung hinter den Gastauscherfasern.

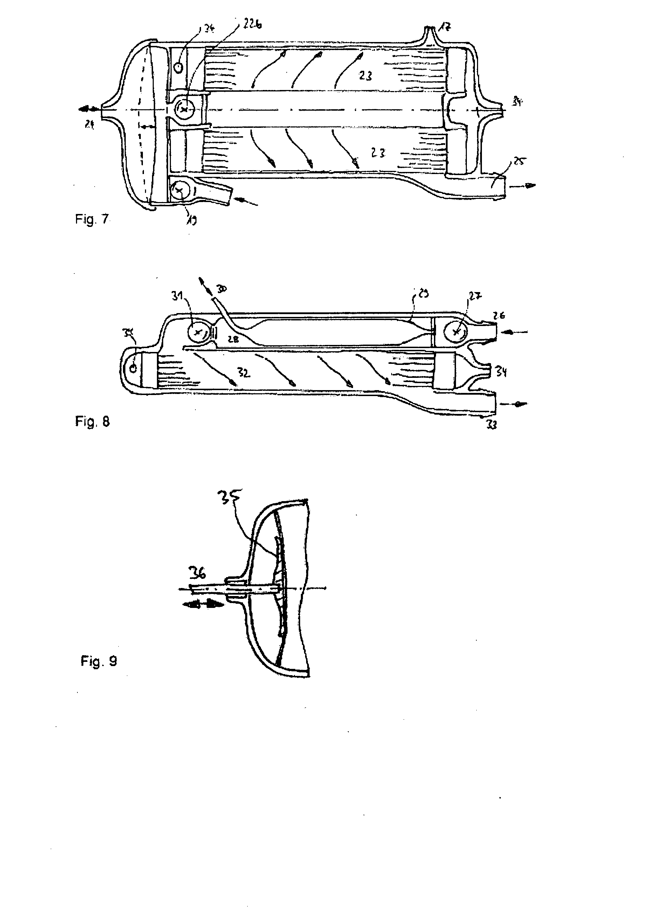

Bei einem weiteren Prinzip (Fig. 6) gelangt das Blut durch ein Ventil 19 in eine Pumpkammer 20, die an einer Seite durch eine flexible Membran 21 begrenzt ist, und durch ein zweites Ventil 22a oder 22b zu den Gastauscherfasern 23. Die Membran ist auf der anderen Seite mit einem Anschlussschlauch 24 verbunden und wird über diesen durch das Antriebsaggregat pulsatil mit Druckluft beaufschlagt und entspannt. Die Gastauscherfasern 23 können tonnenförmig (Fig. 6) oder ringförmig (Fig. 7) angeordnet sein. Während bei der tonnenförmigen Anordnung das Ventil 22a außen sitzt, ist es bei der ringförmigen Anordnung zentrisch angeordnet 22b. Nachdem das Blut bei der tonnenförmigen Faseranordnung diagonal bzw. radial bei der ringförmigen Anordnung den Oxygenationsbereich durchströmt, gelangt es zum tief gelegenen Auslass 23 und von dort zurück zum Patienten. Beim Befüllen des Systems ist ein Entlüftungsanschluss 17 nahe dem Auslass hilfreich. Dieser ist an der höchsten Stelle des Systems angebracht.

Bei einem weiteren Prinzip (Fig. 8) sind die Pumpeinheit und die Gastauschereinheit parallel angeordnet. Das Blut gelangt durch einen Stutzen 26 und über ein Ventil 27 in die Pumpkammer 28, in der sich ein Ballon 29 befindet. Dieser wird über die Zuleitung 30 pulsatil mit Druckluft beaufschlagt und entspannt. Durch ein weiters Ventil 31 gelangt das Blut zu den Gastauscherfasern 32 und von dort über den Auslassstutzen 33 zurück zum Patienten.

Bei allen Lösungen werden die Gastauscherfasern über eine Zu- und Ableitung 34 mit Beatmungsgas versorgt bzw. das Beatmungsgas abgeleitet.

Denkbar ist, dass bei den Lösungen mit Membran (Fig. 6 und 7) diese durch eine Druckplatte 35 und einen Stößel 36 mechanisch angetrieben werden (Fig 9). Hierzu wird eine Antriebskonsole mit einem geeigneten Aktuator verwendet. Dieser Aktuator kann auch pneumatisch ausgeführt werden.

Vorgeschlagen werden für die beschriebenen Lösungen verschiedene Ventilgeometrien. So lässt sich die Erfindung mit Kugelventilen wie dargestellt realisieren. Denkbar sind aber auch Segelventile, Scheibenventile oder Membranventile.

Durch verschiedene geometrische Anordnungen der Pumpkammern, Ventile, Ausführung der Ventile und Oxygenatorfasern in Kombination mit beiden Prinzipien ergeben sich so unterschiedliche Bauformen, die ein äußerst kompaktes ECMO System ermöglichen.

Es wird vorgeschlagen die Blutzu- und Ableitung in einer geometrischen Richtung zu gestalten, um den Anschluss an den Patienten zu vereinfachen und die Anschlusskanülen möglichst kurz zu halten.

Es wird vorgeschlagen das System optional bereits gefüllt herzustellen, zu liefern und zu lagern, damit es rasch einsatzbereit ist.

Die pulsatile Blutförderung wirkt sich vorteilhaft auf den Gasaustausch im Oxygenator und die Auswaschung des Gesamtsystems durch eine kontinuierliche Durchmischung des Blutes und eine verbesserte Auswaschung von kritischen Bereichen aus. So wird der Thrombenbildung entgegengewirkt.

Beide Wirkprinzipien lassen sich zusätzlich zum Gastauscher auch mit einem Wärmetauscher kombinieren.

Da die Pumpenergie lediglich über einen Gasanschluss (mit Ausnahme der letzten Lösung) und nicht mechanisch über einen Elektromotor wie gängig, der mit dem System verbunden ist, übertragen wird, lässt sich das System flexibler und näher am bzw. auf dem Patienten platzieren.

Für den Antrieb der pulsatilen Pumpe ergeben sich verschiedene Optionen, was die Gerätekombination und den Einsatz flexibler gestaltet.

Da für den Gasaustausch im Oxygenator Sauerstoff in komprimierter Form in Gasflaschen oder über eine zentrale Versorgungsleitung zur Verfügung steht, kann dieser Gasdruck genutzt werden, um auch mit Hilfe einer geeigneten pneumatischen Schaltung den pulsatilen Antrieb zu ermöglichen. So wird keine zusätzliche Energiequelle benötigt und es lässt sich ein kompakter, einfacher und preiswerter Antrieb ermöglichen.

## Patentansprüche

1. Anordnung mit einer Blutpumpe (5) und einem Gasaustauscher (7) zur extrakorporalen Membranoxygenierung, wobei die Blutpumpe als pulsierende Blutpumpe ausgebildet ist und mit dem Gasaustauscher im gleichen Gehäuse angeordnet ist, ***dadurch gekennzeichnet, dass*** die pulsatile Blutpumpe und der Gasaustauscher mit der gleichen Gasquelle in Verbindung stehen.

2. Anordnung nach Anspruch 1, ***dadurch gekennzeichnet, dass*** sie eine Blutzuleitung aufweist, die mit der pulsierend ausgebildeten Blutpumpe verbunden ist und eine Blutableitung, die mit dem Gasaustauscher in Verbindung steht, wobei Blutzuleitung und Blutableitung als Kanülen oder Schlauchanschluss ausgebildet sind.

3. Anordnung nach Anspruch 1 oder 2, ***dadurch gekennzeichnet, dass*** sie eine Blutzu-und eine Blutableitung aufweist, die eine Länge von 80 cm oder weniger als Verbindung zu einem Patienten aufweisen.

4. Anordnung nach einem der vorhergehenden Ansprüche, ***dadurch gekennzeichnet, dass*** eine am Gehäuse angeordnete Blutableitung und eine am Gehäuse angeordnete Blutableitung in die gleiche Richtung weisen.

5. Anordnung nach einem der vorhergehenden Ansprüche, ***dadurch gekennzeichnet, dass*** eine Blutzuleitung und eine Blutableitung an der gleichen Seite des Gehäuses angeordnet sind.

6. Anordnung nach einem der vorhergehenden Ansprüche, ***dadurch gekennzeichnet, dass*** die pulsatile Blutpumpe in einer axialen Ausrichtung des Gasaustauschers wirkt

7. Anordnung nach einem der vorhergehenden Ansprüche, ***dadurch gekennzeichnet, dass*** die pulsatile Blutpumpe radial innerhalb des Gasaustauschers angeordnet ist.

8. Anordnung nach einem der vorhergehenden Ansprüche, ***dadurch gekennzeichnet, dass*** die pulsatile Blutpumpe an einer Stirnseite des Gasaustauschers angeordnet ist.

9. Anordnung nach einem der vorhergehenden Ansprüche, ***dadurch gekennzeichnet, dass*** die pulsatile Blutpumpe pneumatisch angetrieben wird.

10. Anordnung nach einem der vorhergehenden Ansprüche, ***dadurch gekennzeichnet, dass*** die pulsatile Blutpumpe als Ballonpumpe ausgeführt ist.

11. Anordnung nach einem der vorhergehenden Ansprüche, ***dadurch gekennzeichnet, dass*** die pulsatile Blutpumpe als Membranpumpe ausgeführt ist, deren Membran derart vorgespannt ist, dass ihre passive Lage die der maximalen Pumpenfüllung ist.

12. Anordnung nach einem der vorhergehenden Ansprüche, ***dadurch gekennzeichnet dass*** der Gasaustauscher über ein Ventil mit pulsierendem Druckgas in Verbindung steht.

13. Anordnung nach einem der vorhergehenden Ansprüche, ***dadurch gekennzeichnet, dass*** der Gasauslass der pulsierenden Blutpumpe mit dem Gasaustauscher in Verbindung steht.

14. Anordnung nach einem der vorhergehenden Ansprüche, ***dadurch gekennzeichnet, dass*** der Gasauslass eines pulsierenden Pumpenantriebs mit dem Gasaustauscher in Verbindung steht.

## Claims

1. An arrangement having a blood pump (5) and a gas exchanger (7) for extracorporeal membrane oxygenation, wherein the blood pump is formed as a pulsating blood pump and is arranged with the gas exchanger in the same housing, ***characterised in that*** the pulsatile blood pump and the gas exchanger are connected to the same gas source.

2. The arrangement according to Claim 1, ***characterised in that*** it has a blood feed line, which is connected to the pulsating blood pump, and a blood discharge line, which is connected to the gas exchanger, wherein the blood feed line and blood discharge line are formed as cannulas or a tube connection.

3. The arrangement according to Claim 1 or 2, ***characterised in that*** it has a blood feed line and a blood discharge line, which have a length of 80 cm of less as connection to a patient.

4. The arrangement according to one of the preceding claims, ***characterised in that*** a blood feed line arranged on the housing and a blood discharge line arranged on the housing point in the same direction.

5. The arrangement according to one of the preceding claims, ***characterised in that*** a blood feed line and a blood discharge line are arranged on the same side of the housing.

6. The arrangement according to one of the preceding claims, ***characterised in that*** the pulsatile blood pump acts in an axial orientation of the gas exchanger.

7. The arrangement according to one of the preceding claims, ***characterised in that*** the pulsatile blood pump is arranged radially within the gas exchanger.

8. The arrangement according to one of the preceding claims, ***characterised in that*** the pulsatile blood pump is arranged on an end face of the gas exchanger.

9. The arrangement according to one of the preceding claims, ***characterised in that*** the pulsatile blood pump is driven pneumatically.

10. The arrangement according to one of the preceding claims, ***characterised in that*** the pulsatile blood pump is formed as a balloon pump.

11. The arrangement according to one of the preceding claims, ***characterised in that*** the pulsatile blood pump is formed as a diaphragm pump, of which the diaphragm is biased in such a way that the passive position thereof is the maximum pump filling.

12. The arrangement according to one of the preceding claims, ***characterised in that*** the gas exchanger is connected via a valve to pulsating pressurised gas.

13. The arrangement according to one of the preceding claims, ***characterised in that*** the gas outlet of the pulsating blood pump is connected to the gas exchanger.

14. The arrangement according to one of the preceding claims, ***characterised in that*** the gas outlet of a pulsating pump drive is connected to the gas exchanger.

## Revendications

1. Agencement comportant une pompe à sang (5) et un échangeur gazeux (7) pour l'oxygénation par membrane extracorporelle, dans lequel la pompe à sang est conçue en tant que pompe à sang pulsatoire et est disposée dans le même logement avec l'échangeur gazeux, ***caractérisé en ce que*** la pompe à sang pulsatoire et l'échangeur gazeux sont en liaison avec la même source de gaz.

2. Agencement selon la revendication 1, ***caractérisé en ce qu'**il* présente un tube d'arrivée de sang qui est relié à la pompe à sang pulsatoire et un tube d'évacuation de sang qui est en liaison avec l'échangeur gazeux, sachant que le tube d'arrivée de sang et le tube d'évacuation de sang sont conçus en tant que canules ou raccord flexible.

3. Agencement selon la revendication 1 ou 2, ***caractérisé en ce qu**'il* présente un tube d'arrivée de sang et un tube d'évacuation de sang qui présentent une longueur de 80 cm ou moins en tant que liaison vers un patient.

4. Agencement selon l'une des revendications précédentes, ***caractérisé en ce qu**'un* tube d'arrivée de sang disposé sur le logement et un tube d'évacuation de sang disposé sur le logement sont orientés dans la même direction.

5. Agencement selon l'une des revendications précédentes, ***caractérisé en ce qu**'un* tube d'arrivée de sang et un tube d'évacuation de sang sont disposés du même côté du logement.

6. Agencement selon l'une des revendications précédentes, ***caractérisé en ce que*** la pompe à sang pulsatoire agit dans un alignement axial de l'échangeur gazeux.

7. Agencement selon l'une des revendications précédentes, ***caractérisé en ce que*** la pompe à sang pulsatoire est disposée radialement à l'intérieur de l'échangeur gazeux.

8. Agencement selon l'une des revendications précédentes, ***caractérisé en ce que*** la pompe à sang pulsatoire est disposée sur une face avant de l'échangeur gazeux.

9. Agencement selon l'une des revendications précédentes, ***caractérisé en ce que*** la pompe à sang pulsatoire est à entraînement pneumatique.

10. Agencement selon l'une des revendications précédentes, ***caractérisé en ce que*** la pompe à sang pulsatoire est conçue en tant que pompe à ballonnet.

11. Agencement selon l'une des revendications précédentes, ***caractérisé en ce que*** la pompe à sang pulsatoire est conçue en tant que pompe à membrane dont la membrane est pré-tendue de sorte que sa position passive soit celle du remplissage maximal de la pompe.

12. Agencement selon l'une des revendications précédentes, ***caractérisé en ce que*** l'échangeur gazeux est en liaison avec du gaz comprimé pulsatoire via une valve.

13. Agencement selon l'une des revendications précédentes, ***caractérisé en ce que*** l'évacuation de gaz de la pompe à sang pulsatoire est en liaison avec l'échangeur gazeux.

14. Agencement selon l'une des revendications précédentes, ***caractérisé en ce que*** l'évacuation de gaz d'un entraînement de pompe pulsatoire est en liaison avec l'échangeur gazeux.
